# EUROPÄISCHE PATENTANMELDUNG

(11) **EP 3 135 325 A1**
(43) Veröffentlichungstag der Anmeldung: **01.03.2017**
(21) Anmeldenummer: 15182115.4
(22) Anmeldetag: 24.08.2015
(51) Int. Cl.: A61M 1/10

(54) **REGELEINRICHTUNG UND VERFAHREN FÜR EINE HERZPUMPE**

(71) Anmelder: Berlin Heart GmbH, 12247 Berlin (DE)
(72) Erfinder: GRANEGGER, Marcus, CH-8057 Zürich (CH); WIESENER, Constantin, 14471 Potsdam (DE); KARCH, Dominik, 12157 Berlin (DE)
(74) Vertreter: Pfenning, Meinig & Partner mbB

(57) **Zusammenfassung**

Die Neuerung betrifft eine Regeleinrichtung (10, 10a, 10b) für eine Herzpumpe (3) mit einer Einrichtung zur Ermittlung des enddiastolischen Fülldrucks (19) in einer Herzkammer und einer Einrichtung (10a) zur Zuordnung einer Förderrate der Pumpe, insbesondere einer Pumpendrehzahl oder einer elektrischen Pumpenleistung, zu dem ermittelten enddiastolischen Fülldruck. Durch die Berücksichtigung des enddiastolischen Fülldrucks wird eine robuste, der physiologischen Regelung ähnliche Betriebsmöglichkeit der Herzpumpe geschaffen.

## Beschreibung

Die Neuerung liegt auf dem Gebiet der Elektrotechnik und lässt sich mit besonderem Vorteil auf dem medizintechnischen Gebiet einsetzen. Konkret befasst sich die Neuerung mit einer Regeleinrichtung für eine Herzpumpe sowie der Erfassung der Messgrößen, die der Regelung zugrunde gelegt werden sollen.

Im Folgenden wird als besonderes Beispiel der Neuerung die Anwendung einer Regelung für eine Rotationspumpe betrachtet, da derartige Pumpen sich bezüglich der Förderleistung besonders einfach steuern lassen. Dies soll jedoch nicht die Anwendung mit anderen bezüglich ihrer Förderleistung steuerbaren Pumpentypen ausschließen.

Oft werden Rotationsblutpumpen mit einer konstanten Drehzahl betrieben, um das Herz des Patienten kontinuierlich zu entlasten. Dies führt jedoch besonders bei körperlicher Betätigung oder Belastung des Patienten oft zu einer Minderversorgung im Patientenkörper und/oder zu einer Überlastung des Herzens. Die körperliche Leistungsfähigkeit des Patienten ist daher beeinträchtigt.

Es werden in der Literatur verschiedene Regelungsmöglichkeiten für derartige Herzpumpen diskutiert, wobei üblicherweise erfasste Betriebsparameter der Pumpe einer Regelung zugrunde gelegt werden. In die Praxis umgesetzt sind diese Möglichkeiten bisher nicht.

Aus der US 6 623 420 B2 ist eine Regeleinrichtung für eine Blutpumpe mit einem Drucksensor in der Herzkammer bekannt. Es wird dort der minimale linksventrikuläre Fülldruck gemessen, und die Steuerung bewirkt, diesen Fülldruck in einem bestimmten Bereich zu halten. Dabei ergibt sich insbesondere das Problem, dass der absolute Regelbereich für den minimalen Ventrikeldruck klein ist, da dieser sich meist in der flachen Region der enddiastolischen Druck/Volumen-Kurve befindet. Eine kleine Änderung des minimalen ventrikulären Drucks müsste, um diesen konstant zu halten, eine große Änderung der Pumpendrehzahl nach sich ziehen. Dies führt auch dazu, dass ein kleiner Fehler in der Druckmessung eine große Auswirkung auf die Änderung der Pumpendrehzahl hat. Da Drucksensoren üblicherweise driftbehaftet sind, kann allein dieser Drift des Sensors zu ungeeigneten Regelungsbereichen der Drehzahl führen.

Zudem besteht der Nachteil, dass gemäß dem Stand der Technik der genannten US-Schrift der minimale Ventrikeldruck auf einen Zielwert geregelt wird. Dies entspricht nicht dem physiologischen Prinzip des Frank-Starling-Effekts. Dieser Mechanismus sorgt für eine Erhöhung des Herzzeitvolumens aufgrund einer Erhöhung der Vorlast. Die Vorlast wird allerdings vom Herzen nicht auf einen Wert geregelt, sondern ist z. B. bei körperlicher Aktivität und hohem Herzzeitvolumen auch stark erhöht. Diese Erhöhung der linksventrikulären Vorlast limitiert auch durch eine Erhöhung der rechtsventrikulären Nachlast das Herzzeitvolumen. Daher balanciert dieser Mechanismus das Herzzeitvolumen des rechten und linken Herzens.

Der vorliegenden Neuerung liegt vor dem Hintergrund des Standes der Technik die Aufgabe zugrunde, eine Regelung der eingangs genannten Art für eine Herzpumpe zu schaffen, die möglichst unempfindlich gegenüber einem Drift von verwendeten Drucksensoren reagiert und möglichst viele Elemente einer physiologischen Regelung aufweist.

Die Aufgabe wird mit den Merkmalen der Neuerung gemäß Patentanspruch 1 gelöst. Die Patentansprüche 2 bis 9 geben vorteilhafte Ausgestaltungen der Neuerung an. Patentanspruch 10 bezieht sich auf ein Computerprogrammprodukt zur Implementierung der Neuerung, Patentanspruch 11 nennt ein Verfahren zur Regelung einer Herzpumpe, und die Patentansprüche 12 und 13 geben besondere Ausgestaltungen des Verfahrens zur Regelung der Herzpumpe an.

Gegenstand dieses Schutzrechts ist außerdem eine Herzpumpe bzw. ein Herzpumpensystem (d. h. Herzpumpe plus Peripherie, wie Stromversorgung etc.), die sämtliche Merkmale der Patentansprüche sowie der folgenden Beschreibung bzw. der Figuren enthalten kann (siehe beispielsweise Patentansprüche 14 und 15).

Der Patentanspruch 1 benennt als Neuerung eine Regeleinrichtung für eine Herzpumpe mit einer Einrichtung zur Ermittlung des enddiastolischen Fülldrucks in einer Herzkammer und einer Einrichtung zur Zuordnung eines Wertes eines Betriebsparameters, insbesondere der Förderrate der Pumpe, weiter insbesondere einer Pumpendrehzahl oder einer elektrischen Pumpenleistung, zu dem ermittelten enddiastolischen Fülldruck.

Bestandteil der Regeleinrichtung ist somit eine Einrichtung zur Ermittlung des enddiastolischen Fülldrucks in einer Herzkammer, die üblicherweise zu diesem Zweck einen Drucksensor aufweist, der nicht nur statisch einzelne Druckwerte messen kann, sondern es ermöglicht, einen Druckverlauf in einer Herzkammer zu ermitteln. Der übliche Druckverlauf des Herzens während der Periode der Herzfrequenz ist bekannt. Es soll aus dem Verlauf der enddiastolische Fülldruck in einer Herzkammer bestimmt werden. Dies ist der Druck, der vor Beginn der Kontraktion der Herzkammer im dortigen Ventrikel nach der diastolischen Phase herrscht. Der enddiastolische Druck hat einen größeren Variationsbereich (ca. 0 bis 30 mmHg) als der minimale Ventrikeldruck und ist daher weniger sensibel für kleine Messfehler oder Sensorendrift.

Aus dem Druckwert, der beim Erreichen des enddiastolischen Fülldrucks erreicht wird, bestimmt die Regeleinrichtung Parameter für den Betrieb der Pumpe, insbesondere eine Förderleistung oder eine anzusteuernde Pumpendrehzahl.

Zur Umsetzung der Neuerung ist insbesondere vorgesehen, dass die Regeleinrichtung mit einem in einer Herzkammer angeordneten Absolutdrucksensor oder einer sonstigen Druckmesseinrichtung verbunden ist. Dabei kann zudem vorgesehen sein, dass eine Einrichtung zur Korrektur des gemessenen Absolutdrucks unter Berücksichtigung eines ermittelten Atmosphärendrucks vorgesehen ist. Durch eine zusätzliche Korrektureinrichtung, beispielsweise mithilfe eines zusätzlichen Drucksensors, der außerhalb des Herzens angeordnet ist, können Druckschwankungen außerhalb des Patientenkörpers, die sich auf die Messung des Fülldrucks auswirken würden, eliminiert werden.

Die neuerungsgemäße Regeleinrichtung zeichnet sich dadurch aus, dass der Zieldruck variabel, insbesondere von der Drehzahl/Förderrate der Pumpe, der Förderrate des Herzens oder einer Verknüpfung beider Größen abhängig ist. Besonders wichtig ist dabei, dass die Förderrate oder Leistung der Pumpe nicht auf Erreichung eines bestimmten Werts des enddiastolischen Fülldrucks gerichtet ist, so dass in Abhängigkeit von den aktuellen und individuellen physiologischen Bedingungen ein bestimmter Zielwert für den enddiastolischen Fülldruck angesteuert werden kann. Dies macht die Integration von Aspekten einer physiologischen Regelung in die neuerungsgemäße möglich.

Beispielsweise kann vorgesehen sein, dass die Regeleinrichtung einen Proportionalregler aufweist. Es kann also mit Absicht kein P/I-Regler (Proportional/ Integral-Regler) vorgesehen sein, der Korrekturmaßnahmen einleiten würde, die von der Entfernung des ermittelten enddiastolischen Fülldrucks von einem Zielwert abhängig wären, so dass nach diesem Verfahren bestimmte feste Fülldruckwerte angesteuert würden. Die physiologische Regelung in einem gesunden Kreislauf funktioniert anders, so dass abhängig von der Belastung verschiedene Werte für den enddiastolischen Fülldruck erreicht werden.

Grundsätzlich soll jedoch die Verwendung eines P/I-Reglers im Rahmen der Neuerung nicht ausgeschlossen werden.

Die vorliegende Neuerung lässt grundsätzlich auch eine Regelung der Pumpe zu, die auf das Erreichen eines bestimmten Zieldruckwerts durch den enddiastolischen Druck einer Herzkammer gerichtet ist.

Im Einzelnen kann die Regeleinrichtung derart gestaltet sein, dass die Regeleinrichtung eine Speichereinrichtung aufweist, in der verschiedenen Bereichen des ermittelten enddiastolischen Fülldrucks jeweils eine bestimmte Reglerverstärkung zugeordnet ist.

Es kann auch vorgesehen sein, dass die Regeleinrichtung eine Speichereinrichtung aufweist, in der verschiedenen Werten des ermittelten enddiastolischen Fülldrucks jeweils ein die Förderrate der Pumpe charakterisierender Wert, insbesondere eine Drehzahl oder eine elektrische Leistung der Pumpe oder ein Wert eines anderen Betriebsparameters der Pumpe, zugeordnet ist.

Dabei kann zudem gemäß einer Ausführungsform der Neuerung vorgesehen sein, dass in der Speichereinrichtung zwischen den Druckwerten und Reglerverstärkungen und/oder Drehzahlen und/oder Förderraten der Pumpe ein nichtlinearer Zusammenhang besteht.

Im einfachsten Fall kann die Neuerung durch ein Computerprogrammprodukt mit einem Programm, das zu einem ermittelten enddiastolischen Fülldruck in einer Herzkammer eine Drehzahl/Förderrate einer Pumpe berechnet und zuordnet und die Pumpe mit dieser Förderrate ansteuert, implementiert sein. Das Computerprogrammprodukt kann selbstverständlich auch so ausgestaltet sein, dass es die im Folgenden beschriebenen Verfahren zur Regelung einer Herzpumpe implementiert.

Die Neuerung bezieht sich außer auf eine Regeleinrichtung der oben genannten Art und ein Computerprogrammprodukt auch auf ein Verfahren zur Regelung einer Herzpumpe unter Berücksichtigung von Messwerten des Blutdrucks in einer Herzkammer, wobei der Regelung der enddiastolische Fülldruck in der Herzkammer zugrunde gelegt wird und insbesondere der Druck in der Herzkammer mittels eines Absolutdrucksensors gemessen wird.

Eine besondere Ausgestaltung des Verfahrens sieht dabei vor, dass der enddiastolische Fülldruck als Zielgröße der Regelung variabel ist und insbesondere von der Förderrate des Herzens oder der Drehzahl/Förderrate der Pumpe oder einer Verknüpfung beider Werte abhängt. Hierzu kann insbesondere vorgesehen sein, dass die Regelung den Zieldruck unter Berücksichtigung der Förderrate des Herzens oder der Pumpe oder der Drehzahl der Pumpe oder einer Verknüpfung dieser Werte ermittelt.

Eine Implementierung sieht hierzu vor, dass die Regelung eine Proportionalregelung ist. Insbesondere kann die Regelung ohne einen Integralregler ausgeführt sein.

Es kann in einer weiteren Implementierung des Verfahrens auch vorgesehen sein, dass die Regelung mittels einer Speichereinrichtung jeweils einem gemessenen oder ermittelten enddiastolischen Druckwert einen die Förderrate der Pumpe charakterisierenden Wert, insbesondere eine Pumpendrehzahl oder eine Pumpenleistung, zuordnet.

Zudem kann auch vorgesehen sein, dass die Regeleinrichtung eine Speichereinrichtung aufweist, in der verschiedenen Bereichen des ermittelten enddiastolischen Fülldrucks jeweils eine Reglerverstärkung zugeordnet ist.

Eine weitere Implementierungsform der Neuerung kann vorsehen, dass in der Speichereinrichtung einer Mehrzahl von Druckwerten jeweils Förderraten der Pumpe oder andere Betriebsparameterwerte der Pumpe gemäß einem insbesondere nichtlinearen Zusammenhang zugeordnet sind.

Im Folgenden werden Ausführungsbeispiele der Neuerung anhand von Figuren einer Zeichnung gezeigt und nachfolgend erläutert. Dabei zeigt
- Fig. 1: schematisch eine Herzpumpeneinrichtung mit einer Regeleinrichtung,
- Fig. 2: schematisch in einem Ablaufdiagramm das neuerungsgemäße Regelungskonzept,
- Fig. 3: schematisch in einem Diagramm den Druckverlauf während einer Herzperiode sowie
- Fig. 4: schematisch die Darstellung des Drucks und des ventrikulären Volumens während der Herzperiode in drei Standardfällen mit unterschiedlichem enddiastolischem Fülldruck

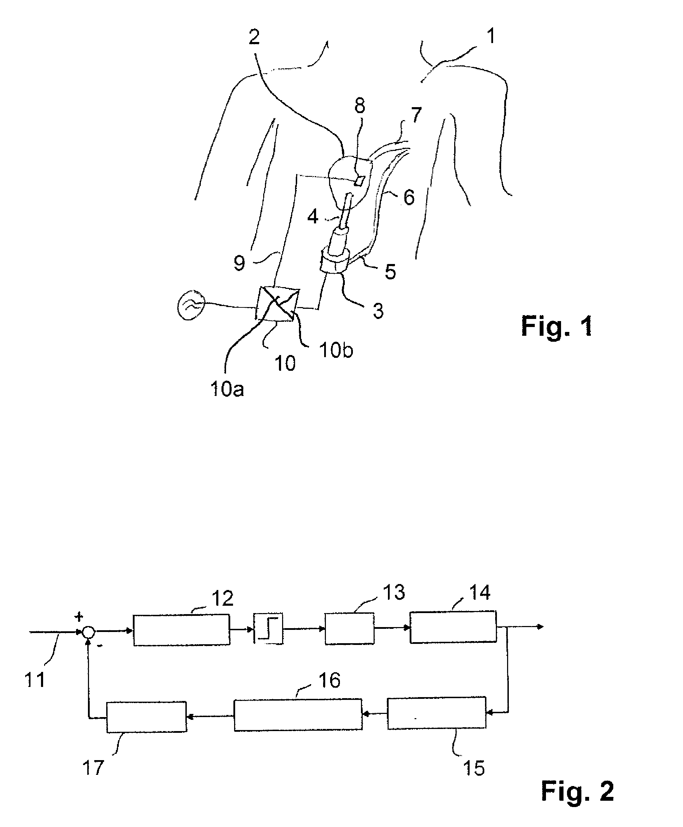

Figur 1 zeigt schematisch den Körper 1 eines Patienten sowie das zu unterstützende Herz 2 und eine am Herzen angeordnete Rotationspumpe 3. Die Pumpe 3 ist mittels einer Einlasskanüle 4 mit dem Herzen 2 des Patienten verbunden und saugt Blut aus dem rechten Ventrikel ab, um dieses zum Pumpenauslass 5 und durch die Auslasskanüle 6 in die Aorta 7 zu fördern.

Als Element der Regeleinrichtung ist ein Drucksensor 8 im Innenraum des Herzens vorgesehen, der über eine Leitung 9 mit einer Verarbeitungseinrichtung 10 der Regeleinrichtung verbunden ist. Die Verarbeitungseinrichtung 10 steuert elektrisch die Pumpe 3 unter Berücksichtigung von Resultaten der Druckmessung an. Dazu ordnet eine Einrichtung 10a zunächst einem enddiastolischen Fülldruck einen Wert eines Betriebsparameters der Pumpe, beispielsweise eine Förderrate der Pumpe oder eine Pumpendrehzahl, zu. Hierzu ist die Einrichtung 10a mit einer Speichereinrichtung 10b verbunden.

Ein Problem dabei ist die sich ändernde Anforderung an den Blutdurchsatz im Körper des Patienten. Üblicherweise wird bei steigender körperlicher Belastung an vielen Stellen des Körpers des Patienten eine intensivere Durchblutung benötigt, was dazu führt, dass über das venöse System mehr Blut zum Herzen zurückgeliefert wird (venous return). Eine physiologische Regelung eines Herzunterstützungssystems muss Maßnahmen vorsehen, um das vom rechten Herzen gelieferte Blutvolumen wegzupumpen. Im physiologisch ohne Unterstützung funktionierenden Herzen wird bei einer Erhöhung des end-diastolischen ventrikulären Drucks der Auswurf des Ventrikels erhöht, um das vermehrt angelieferte Blutvolumen wegpumpen zu können (Frank-Starling-Effekt). Eine ähnliche Regelung kann mittels der vorgestellten Neuerung nachgebildet werden, d. h. eine Regelung, die nicht gezielt den enddiastolischen Fülldruck konstant hält, aber trotzdem auf Änderungen des enddiastolischen Fülldrucks reagiert.

Hierzu soll zunächst grundsätzlich der Mechanismus der Regelung anhand der Figur 2 beschrieben werden. Figur 2 beschreibt einen Regelkreis, bei dem am Eingang 11 ein Zielwert für den enddiastolischen Fülldruck vorgegeben wird. Ein Mikrocontroller, der in der Verarbeitungseinrichtung 10 angeordnet ist, gibt in einem Verfahrensschritt 12 in Abhängigkeit vom aktuellen enddiastolischen Fülldruck als Parameter der Pumpe beispielsweise die Drehzahl der Förderrate vor. Der Controller weist eine Speichereinrichtung auf, in der der Solldrehzahl der Pumpe oder einer Sollförderrate eine bestimmte Ansteuergröße, wie beispielsweise eine Stromstärke oder eine andere elektrische Steuergröße, zugeordnet wird, mit der die Pumpe angesteuert wird (Schritt 13). Darauf stellt sich im Ventrikel ein bestimmter Druck ein (Schritt 14). Dieser wird durch eine Sensormessung 15 laufend gemessen, und aus dem Druckverlauf wird in einem Verfahrensschritt 16 der enddiastolische Fülldruck der periodischen Herzaktivität identifiziert. Es kann dann in einem Filterschritt 17 eine Signalglättung stattfinden, und der tatsächlich erreichte enddiastolische Fülldruck wird gemeinsam mit dem Zieldruck zum Eingang 11 der Regelschleife gegeben.

Figur 2 macht keine Angaben über die Natur der Regelung; beispielsweise muss nicht vorgesehen sein, dass die Regelung die Differenz zwischen dem tatsächlich ermittelten enddiastolischen Fülldruck und dem Zielwert des enddiastolischen Fülldrucks minimiert. Es kann jedoch die Abweichung des aktuellen enddiastolischen Fülldrucks vom Zielwert im Controller zu bestimmten Ansteuerungswerten der Pumpe verarbeitet werden.

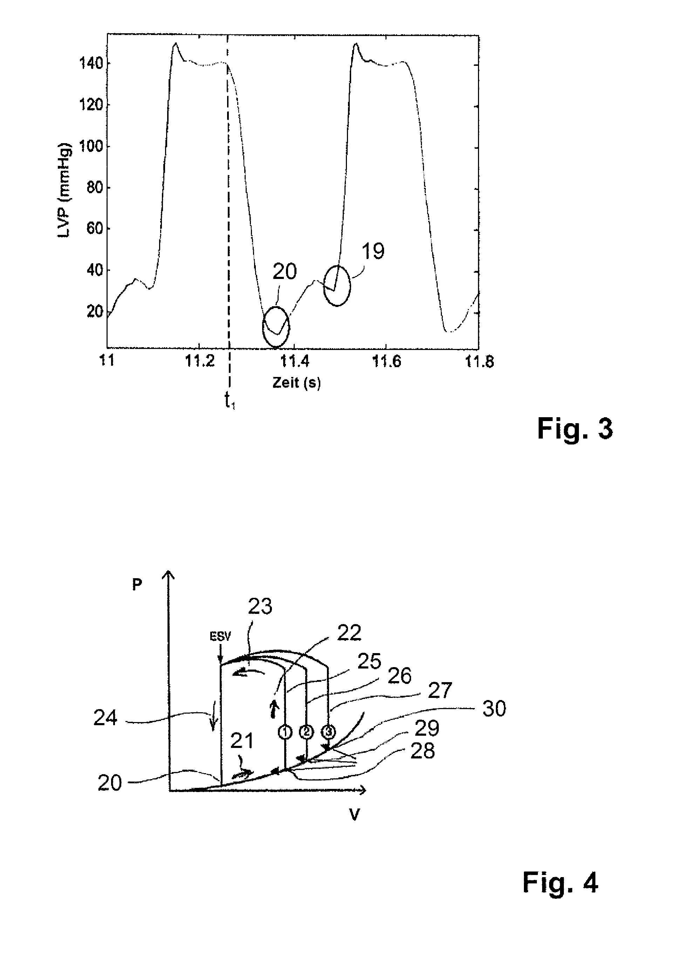

Anhand der Figur 3 soll grundsätzlich der Druckverlauf im linken Ventrikel schematisch dargestellt werden. Die Zeit in Sekunden ist dabei auf der horizontalen Achse aufgetragen, während der Druck in Millimetern Quecksilbersäule auf der vertikalen Achse aufgetragen ist. Die Betrachtung soll zum Zeitpunkt t₁ kurz nach 11,2 Sekunden begonnen werden, wenn der Druck im linken Ventrikel abfällt, d. h. der Ventrikel relaxiert. Nach der aktiven Relaxationsphase beginnt die Füllungsphase des Ventrikels, und der Ventrikeldruck erreicht sein absolutes Minimum, das durch den ersten Markierungskreis 18 hervorgehoben ist. Füllt sich der Ventrikel, so steigt der ventrikuläre Druck langsam, bis der linke Ventrikel in der Systole kontrahiert. Am Beginn der Systole, in der isovolumetrischen Kontraktionsphase, steigt der Druck schlagartig an. Am Übergang zwischen dem langsamen Anstieg in der Diastole und dem schnellen Anstieg in der Systole befindet sich der enddiastolische Fülldruck, markiert durch den Kreis 19.

Bei einer Änderung der physiologischen Belastung des Patienten verändert sich sowohl der minimale diastolische Druck 18 als auch der enddiastolische Fülldruck 19. Die Erfahrung zeigt, dass sich der enddiastolische Fülldruck 19 in absoluten Einheiten stärker ändert als der minimale diastolische Druck, so dass eine Regelung, die am enddiastolischen Fülldruck ansetzt, wesentlich unempfindlicher ist als eine Regelung, die jeweils von der Ermittlung des minimalen diastolischen Drucks ausgeht. Sensordriften und Messfehler werden auf diese Weise deutlich schwächer gewichtet.

Figur 4 zeigt beispielhaft und schematisch, wie die angestrebte Regelung analog zu einem physiologisch geregelten Herzen wirkt. Auf der horizontalen Achse ist dazu das Füllvolumen des linken Ventrikels aufgetragen, während auf der vertikalen Achse der Druck im Ventrikel aufgetragen ist. In einem ersten Fall strömt ausgehend vom Punkt 20 des Graphen Blut in den linken Ventrikel, so dass Volumen und Druck sich in Richtung des Pfeils 21 bis zum Punkt 28 ändern. Der Punkt 28 bezeichnet bei dem gegebenen Volumen den enddiastolischen Fülldruck. Von dort aus wird entlang der Linie 25 in Richtung des Pfeils 22 der Druck erhöht und das Volumen entlang des Pfeils 23 durch Ausstoßen des Blutes reduziert. Danach fällt der Druck entlang des Pfeils 24 wieder zum Anfangspunkt 20.

Wird nun mehr Blut vom Rechtsherzsystem in Richtung des linken Herzens geliefert, so wird das Volumen bis zum Punkt 29 erhöht, was auch einem etwas erhöhten enddiastolischen Druck entspricht. Entsprechend wird entlang der Linie 26 der Druck erhöht und ein größeres Volumen entlang des Pfeils 23 aus dem linken Ventrikel gepumpt.

Dasselbe gilt für ein weiter vergrößertes Blutvolumen im linken Ventrikel, wenn dieses bis zum Punkt 30 gefüllt wird. In diesem Fall wird entlang der Linie 27 der Druck erhöht und ein noch weiter vergrößertes Ausstoßvolumen (Frank-Starling-Effekt) aus dem Ventrikel herausgepresst.

Durch die gezeigten Beispiele soll dargestellt werden, dass das physiologische Regelsystem keineswegs auf einen konstanten enddiastolischen Fülldruck regelt, sondern auf diesen reagiert. Zwischen dem Ausstoßvolumen und dem enddiastolischen Fülldruck gibt es eine Korrelation, so dass der entsprechende Regelungsmechanismus als P-Regler bezeichnet werden kann. Eine derartige Regelung kann mithilfe der vorgestellten Neuerung ähnlich nachgebildet werden.

Eine optimale Entleerung des linken Ventrikels kann mit echokardiographischen Mitteln bestimmt werden, um die Regeleinrichtung optimal einzustellen. Wird eine optimale Förderrate in Form einer Drehzahl der Pumpe beispielsweise mit 7500 Umdrehungen pro Minute ermittelt und ergibt sich bei diesem Betrieb ein enddiastolischer Fülldruck von 12,5 mmHg, dann kann beispielsweise die Regelkurve derart eingestellt werden, dass pro zusätzlichen Millimeter Quecksilbersäule, den der enddiastolische Fülldruck wächst, die Drehzahl der Pumpe um 200 Umdrehungen pro Minute oder einen anderen vorgegebenen Wert heraufgesetzt wird. Entsprechendes sollte bei verringerten Werten des enddiastolischen Fülldrucks gelten, wobei der Zusammenhang zwischen den Zuwächsen des enddiastolischen Fülldrucks und den Änderungen der Pumpendrehzahl nichtlinear sein kann. Es sollte dabei versucht werden, den Betriebsbereich in Bezug auf die Drehzahlen der Pumpe möglichst weit ausnutzen zu können.

Eine Einstellung der Regelung kann über eine echokardiographische Vermessung des Fördervolumens des Ventrikels in regelmäßigen Abständen vorgenommen werden.

Somit könnte die Neuerung eine quasiphysiologische Regelung der Förderleistung einer Blutpumpe ermöglichen und damit eine höhere körperliche Belastbarkeit eines Patienten bzw. eine erhöhte Lebensqualität erreichen.

## Patentansprüche

1. Regeleinrichtung für eine Herzpumpe (3) mit einer Einrichtung (8, 10) zur Ermittlung des enddiastolischen Fülldrucks in einer Herzkammer und einer Einrichtung (10a) zur Zuordnung eines Betriebsparameters, insbesondere der Förderrate der Pumpe, weiter insbesondere einer Pumpendrehzahl oder einer elektrischen Pumpenleistung, zu dem ermittelten enddiastolischen Fülldruck.

2. Regeleinrichtung nach Anspruch 1, **dadurch gekennzeichnet, dass** die Regeleinrichtung mit einem in einer Herzkammer angeordneten Absolutdrucksensor (8) oder einer sonstigen Druckmesseinrichtung verbunden ist.

3. Regeleinrichtung nach Anspruch 1 oder 2, **dadurch gekennzeichnet, dass** eine Einrichtung zur Korrektur des gemessenen Absolutdrucks unter Berücksichtigung eines ermittelten Atmosphärendrucks vorgesehen ist.

4. Regeleinrichtung nach Anspruch 1 oder einem der folgenden, **gekennzeichnet durch** einen variablen, insbesondere von der Drehzahl/Förderrate der Pumpe, der Förderrate des Herzens oder einer Verknüpfung beider Größen abhängigen Zieldruck.

5. Regeleinrichtung nach Anspruch 4, **dadurch gekennzeichnet, dass** der Zieldruck der enddiastolische Druck einer Herzkammer ist.

6. Regeleinrichtung nach Anspruch 1 oder einem der folgenden, **dadurch gekennzeichnet, dass** die Regeleinrichtung einen Proportionalregler aufweist.

7. Regeleinrichtung nach Anspruch 1 oder einem der folgenden, **dadurch gekennzeichnet, dass** die Regeleinrichtung (10) eine Speichereinrichtung (10b) aufweist, in der verschiedenen Bereichen des ermittelten enddiastolischen Fülldrucks jeweils eine Reglerverstärkung zugeordnet ist.

8. Regeleinrichtung nach Anspruch 1 oder einem der folgenden, **dadurch gekennzeichnet, dass** die Regeleinrichtung eine Speichereinrichtung (10b) aufweist, in der verschiedenen Werten des ermittelten enddiastolischen Fülldrucks (19) jeweils ein die Förderrate der Pumpe (3) charakterisierender Wert, insbesondere eine Drehzahl oder eine elektrische Leistung der Pumpe, zugeordnet ist.

9. Regeleinrichtung nach Anspruch 7 oder 8, **dadurch gekennzeichnet, dass** in der Speichereinrichtung (10b) zwischen den Druckwerten und Reglerverstärkungen und/oder Drehzahl und/oder Förderraten der Pumpe (3) ein nichtlinearer Zusammenhang besteht.

10. Computerprogrammprodukt mit einem Programm, das zu einem ermittelten enddiastolischen Fülldruck in einer Herzkammer eine Drehzahl/Förderrate einer Pumpe (3) berechnet und zuordnet und die Pumpe mit dieser Förderrate ansteuert.

11. Verfahren zur Regelung einer Herzpumpe unter Berücksichtigung von Messwerten des Blutdrucks in einer Herzkammer, **dadurch gekennzeichnet, dass** der Regelung der enddiastolischen Fülldruck (19) in der Herzkammer zugrunde gelegt wird und dass insbesondere der Druck in der Herzkammer mittels eines Absolutdrucksensors (8) gemessen wird.

12. Verfahren nach Anspruch 11, **dadurch gekennzeichnet, dass** der enddiastolische Fülldruck (19) als Zielgröße der Regelung variabel ist und insbesondere von der Förderrate des Herzens oder der Drehzahl/Förderrate der Pumpe (3) oder einer Verknüpfung beider Werte abhängt,
wobei vorzugsweise die Regelung den Zieldruck unter Berücksichtigung der Förderrate des Herzens (3) oder der Pumpe oder der Drehzahl der Pumpe oder einer Verknüpfung dieser Werte ermittelt.

13. Verfahren nach Anspruch 12, **dadurch gekennzeichnet, dass** die Regelung eine Proportionalregelung ist und/oder
dass die Regeleinrichtung (10) mittels einer Speichereinrichtung (10b) jeweils einem gemessenen enddiastolischen Druckwert (19) einen die Förderrate der Pumpe (3) charakterisierenden Wert, insbesondere eine Pumpendrehzahl oder eine Pumpenleistung, zuordnet und/oder
dass die Regeleinrichtung (10) eine Speichereinrichtung (10b) aufweist, in der verschiedenen Bereichen des ermittelten enddiastolischen Fülldrucks jeweils eine Reglerverstärkung zugeordnet ist, wobei vorzugsweise
in der Speichereinrichtung (10b) einer Mehrzahl von Druckwerten jeweils Förderraten der Pumpe gemäß einem insbesondere nichtlinearen Zusammenhang zugeordnet sind.

14. Herzpumpe oder Herzpumpensystem, enthaltend eine Regeleinrichtung nach einem der Ansprüche 1 bis 9.

15. Herzpumpe oder Herzpumpensystem, eingerichtet zur Steuerung durch ein Computerprogrammprodukt nach Anspruch 10 und/oder eingerichtet zum Betrieb eines Verfahrens nach einem der Ansprüche 11 bis 13.
